# EUROPEAN PATENT APPLICATION

(11) **EP 4 576 104 A1**
(43) Date of publication of application: **25.06.2025**
(21) Application number: 23854753.3
(22) Date of filing: 12.07.2023
(51) Int. Cl.: G16C 60/00

(54) **MATERIAL DESIGNING DEVICE, MATERIAL DESIGNING METHOD, AND PROGRAM**

(30) Priority: 17.08.2022 JP 2022130049
(71) Applicant: Resonac Corporation, Tokyo 105-7325 (JP)
(72) Inventor: KAKUDA, Kohsuke, Tokyo 105-8518 (JP); TAKEMOTO, Shimpei, Tokyo 105-8518 (JP); OKUNO, Yoshishige, Tokyo 105-8518 (JP)
(74) Representative: Strehl & Partner mbB
(86) International application number: PCT/JP2023/025711
(87) International publication number: WO 2024/038716

(57) **Abstract**

A local search is performed which is unlikely to fall into a local solution, while maintaining initial value dependence. A material designing device is provided including a nearby composition generating unit configured to generate a nearby composition in which each factor included in a composition candidate of a target substance is varied within a predetermined search range; a property predicting unit configured to predict a material property of the target substance based on the nearby composition; a composition candidate updating unit configured to update the composition candidate with the nearby composition, when there is the nearby composition which improves the material property; a search range expanding unit configured to expand the search range, when there is no nearby composition which improves the material property; and a factor switching unit configured to switch a factor of the composition candidate, when the search range exceeds a predetermined maximum search range.

## Description

### FIELD OF THE INVENTION

The present disclosure relates to a material designing device, a material designing method, and a program.

In recent years, technology to improve the efficiency of material development by utilizing machine learning or the like has been used. Material development utilizing machine learning or the like is performed to efficiently search for the optimum solution of a property value by combining a large-scale search and a local search.

### BACKGROUND ART

For example, Patent Document 1 discloses an invention in which a Bayesian model modeling a problem of searching for a combination of values of a plurality of parameters, to give an optimum value of a property value relating to a target substance, is generated, and a search for the combination using the Bayesian model is performed in a search space narrower than the total search space of all combinations of values of the plurality of parameters.

### Related Art Document

### Patent Document

Patent Document 1: Japanese Laid-Open Patent Publication No. 2020-187642

### SUMMARY OF THE INVENTION

### MEANS FOR SOLVING THE PROBLEM

However, in the related art, there is a problem that factor switching is not performed in the local search. If factor switching is not performed, there is a concern of falling into a local solution depending on the initial value in the local search. On the other hand, if random factor switching is performed, there is a concern that a material property which was good at the initial value will be lost.

An object of an aspect of the present disclosure is to perform a local search for which it is difficult to fall into a local solution, while maintaining the initial value dependence.

### [Means for Solving Problems]

The present disclosure has the following structure.

[1] A material designing device comprising: a nearby composition generating unit configured to generate a nearby composition in which each factor included in a composition candidate of a target substance is varied within a predetermined search range; a property predicting unit configured to predict a material property of the target substance based on the nearby composition; a composition candidate updating unit configured to update the composition candidate with the nearby composition, when there is the nearby composition which improves the material property; a search range expanding unit configured to expand the search range, when there is no nearby composition which improves the material property; and a factor switching unit configured to switch a factor of the composition candidate, when the search range exceeds a predetermined maximum search range.
[2] The material designing device according to [1] above, wherein the property predicting unit is configured to predict the material property by using a prediction model in which the material composition of the target substance is an explanatory variable and the material property is an objective variable, and the factor switching unit is configured to switch factors of the composition candidate based on a contribution of each factor in the prediction model.
[3] The material designing device according to [2] above, wherein the property predicting unit is configured to predict a plurality of material properties, and the composition candidate updating unit is configured to update the composition candidate when a first material property is maintained and a second material property is improved.
[4] The material designing device according to [3] above, wherein the factor switching unit is configured to switch a factor having a high contribution to the second material property with a factor having a low contribution to the first material property.
[5] The material designing device according to [4] above, further comprising an initial composition determining unit configured to determine the material composition having a good first material property as the composition candidate, among material compositions generated randomly or with a predetermined increment size from all of material compositions of the target substance.
[6] The material designing device according to [4] or [5] above, wherein the factor switching unit is configured to switch factors of a same type.
[7] The material designing device according to any one of [1] to [6 above, wherein the composition candidate updating unit is configured to output the composition candidate, when the composition candidate has been updated a predetermined number of times.
[8] The material designing device according to any one of [1] to [7] above, wherein the property predicting unit is configured to predict the material property for the nearby composition having a degree of similarity with the composition candidate equal to or greater than a predetermined threshold value.
[9] A material designing method in which a computer executes the following: a procedure of generating a nearby composition in which each factor included in a composition candidate of a target substance is varied within a predetermined search range; a procedure of predicting a material property of the target substance based on the nearby composition; a procedure of updating the composition candidate with the nearby composition, when there is the nearby composition which improves the material property; a procedure of expanding the search range when there is no nearby composition which improves the material property; a procedure of and switching a factor of the composition candidate, when the search range exceeds a predetermined maximum search range.
[10] A program causing a computer to execute the following: a procedure of generating a nearby composition in which each factor included in a composition candidate of a target substance is varied within a predetermined search range; a procedure of predicting a material property of the target substance based on the nearby composition; a procedure of updating the composition candidate with the nearby composition, when there is the nearby composition which improves the material property; a procedure of expanding the search range when there is no nearby composition which improves the material property; and a procedure of switching a factor of the composition candidate, when the search range exceeds a predetermined maximum search range.

According to one aspect of the present disclosure, it is possible to perform a local search for which it is unlikely to fall into a local solution, while maintaining the initial value dependence.

### BRIEF DESCRIPTION OF THE DRAWINGS

[FIG. 1] FIG. 1 is a block diagram showing an example of the overall configuration of the design support system.
[FIG. 2] FIG. 2 is a block diagram showing an example of the hardware configuration of a computer.
[FIG. 3] FIG. 3 is a block diagram showing an example of the functional configuration of the design support system.
[FIG. 4] FIG. 4 is a flowchart showing an example of the processing procedure of the material designing method.
[FIG. 5] FIG. 5(A) is a diagram showing an example of the contribution to the adhesive force. FIG. 5(B) is a diagram showing an example of the contribution to the strength.
[FIG. 6] FIG. 6(A) shows an example of an initial value of a composition candidate. FIG. 6(B) shows an example of a composition candidate after the first factor switching. FIG. 6(C) shows an example of a composition candidate after the second factor switching.
[FIG. 7] FIG. 7(A) shows an example of the first local search. FIG. 7(B) shows an example of the first local search. FIG. 7(C) shows an example of the second local search. FIG. 7(D) shows an example of the second local search.
[FIG. 8] FIG. 8(A) shows an example of the x-th local search. FIG. 8(B) shows an example of the x-th local search. FIG. 8(C) shows an example of the x-th local search. FIG. 8(D) shows an example of the x-th local search.
[FIG. 9] FIG. 9(A) shows an example of the results of the first local search. FIG. 9(B) shows an example of the results of the second local search. FIG. 9(C) shows an example of the results of the third local search. FIG. 9(D) shows an example of the results of the first local search after factor switching. FIG. 9(E) shows an example of the results of the second local search after factor switching. FIG. 9(F) shows an example of the results of the third local search after factor switching.
[FIG. 10] FIG. 10(A) shows an example of the results of the first local search after the first update. FIG. 10(B) shows an example of the results of the first local search after the second update. FIG. 10(C) shows an example of the results of the first local search after the third update.
[FIG. 11] FIG. 11(A) shows an example of a graph summarizing search results from the first search to the last search. FIG. 11(B) shows an example of a graph plotting material properties of the surrounding composition in the first search and the last search.

### DETAILED DESCRIPTION OF THE INVENTION

### [Description of Embodiments of the Present Invention]

Each embodiment of the present invention will be described below with reference to the attached drawings. In the present specification and the drawings, components having substantially the same functional configuration will be denoted by the same reference numerals, and duplicate descriptions will be omitted.

### [Embodiments]

Conventionally, material development has been conducted based on the experience and intuition of engineers. In recent years, technologies to improve the efficiency of material development utilizing machine learning or the like have been used. In material development utilizing machine learning, for example, a large-scale search by machine learning is carried out. According to the large-scale search by machine learning, it is possible to search for material design conditions including areas not expected by humans.

In a large-scale search by machine learning, it is possible to achieve the target value for a given material property A, but it is possible to obtain design conditions that cannot satisfy the standard for another material property B, which has a trade-off relationship with material property A. In this case, since the target value is achieved for the material property A, it is efficient if the design conditions thereof can be utilized to advance the material design.

In this case, for example, it is possible to re-execute the search by machine learning. However, in this other search using machine learning, it is not possible to consider the design conditions that at least achieved the target value for material property A. Moreover, even if a good material property is predicted to be obtained only by the search using machine learning, it is not guaranteed that the material composition is actually feasible.

Also, for example, it is thought of to carry out the search by design of experiments using the obtained design conditions as initial values. Or, it is also thought of that an engineer tunes the obtained design conditions. However, the methods by design of experiments, tuning, etc. have problems such as a large number of experiments and difficulty in considering factors other than those included in the initial values.

In the search using machine learning, an efficient search is sometimes performed by combining large-scale searches and local searches. However, since the factor switching is not performed during the search in the conventional technology, there is a possibility that a local solution depends on the initial value. Even if the factor switching by mutation based on a genetic algorithm is combined, there is a possibility that factor switching unrelated to the material property to be optimized is performed, and the material property may deteriorate.

One embodiment of the present disclosure is a design support system that supports the design of a material to be manufactured using multiple materials. Hereinafter, a material to be designed is called a "target substance", and a material used to manufacture the target substance is called a "material substance". The design support system in the present embodiment is an information processing system that searches for a material composition of a target substance with optimized desired material properties in response to a user's instruction and presents the search result to the user.

The target substance in the present embodiment is, for example, a composite material, such as a material for semiconductors, which is composed of a plurality of resins, additives, and/or fillers. Therefore, the material substance in the present embodiment is, for example, resins, additives, and/or fillers. An example of a material for semiconductors is a resist material, an adhesive, a tackifier, and a sealing material. The target substance and the material substance are not limited thereto, and the present embodiment can be applied to any material produced by using a plurality of materials.

The design support system in the present embodiment uses the material composition of the target substance selected by the user for the initial value of the composition candidate, and repeats a local search in the region nearby the composition candidate to search for a composition candidate of the target substance that can obtain good material properties. In the local search, a plurality of nearby compositions are generated by varying each factor of the composition candidate in a predetermined search range, and if there is a nearby composition whose material properties predicted based on each nearby composition are better than the target value specified by the user, the composition candidate is updated with the nearby composition.

In the design support system of the present embodiment, when there is no nearby composition that improves the material properties in a certain search range, the search range is expanded and a local search is performed again. When the search range exceeds the predetermined maximum search range, the factors of the composition candidates are switched and a local search is performed again. In the factor switching, the factors that have a large influence on the material properties to be improved are switched for the factors that have a small influence on the material properties to be maintained.

Therefore, according to the design support system of the present embodiment, a search result that does not deviate greatly from the initial value of the composition candidate can be obtained with the initial value dependence maintained. In addition, according to the design support system of the present embodiment, the factor switching is performed when no update of the composition candidate occurs within the predetermined search range, and so a search that is not likely to fall into a local solution can be performed.

### <Overall Configuration of the Design Support System>

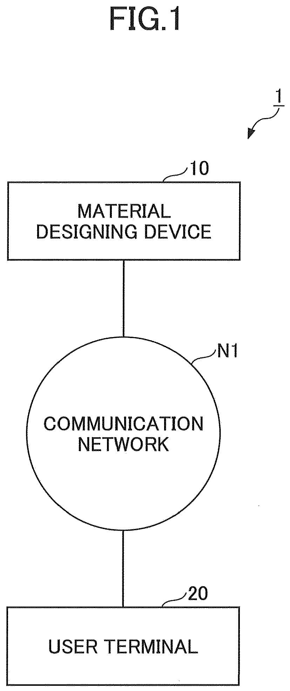

The overall configuration of a design support system of the present embodiment will be described with reference to FIG. 1. FIG. 1 is a block diagram showing an example of the overall configuration of the design support system of the present embodiment.

As shown in FIG. 1, the design support system 1 of the present embodiment includes a material designing device 10 and a user terminal 20. The material designing device 10 and the user terminal 20 are connected so as to enable data communication via a communication network N1 such as a LAN (Local Area Network) or the Internet.

The material designing device 10 is an information processing device such as a personal computer, a workstation, or a server that searches for a material composition of a target substance that can obtain good material properties, in response to a request from the user terminal 20. The material designing device 10 receives initial composition data related to the material composition of the target substance from the user terminal 20. The material designing device 10 searches for a material composition of the target substance based on the initial composition data, and transmits search result data representing the material composition of the target substance, obtained as a search result, to the user terminal 20.

The user terminal 20 is an information processing terminal such as a personal computer, a tablet terminal, or a smartphone operated by a user. The user terminal 20 generates the initial composition data according to an operation by the user, and transmits this initial composition data to the material designing device 10. The user terminal 20 displays the search result to the user, based on the search result data received from the material designing device 10.

The overall configuration of the design support system 1 shown in FIG. 1 is an example, and various system configuration examples can be provided depending on the application and objective. For example, the material designing device 10 may be implemented by a plurality of computers, or as a cloud computing service. Further, for example, the design support system 1 may be implemented by a stand-alone information processing apparatus that combines the functions of the material designing device 10 and the user terminal 20.

### <Hardware Configuration of the Design Support System>

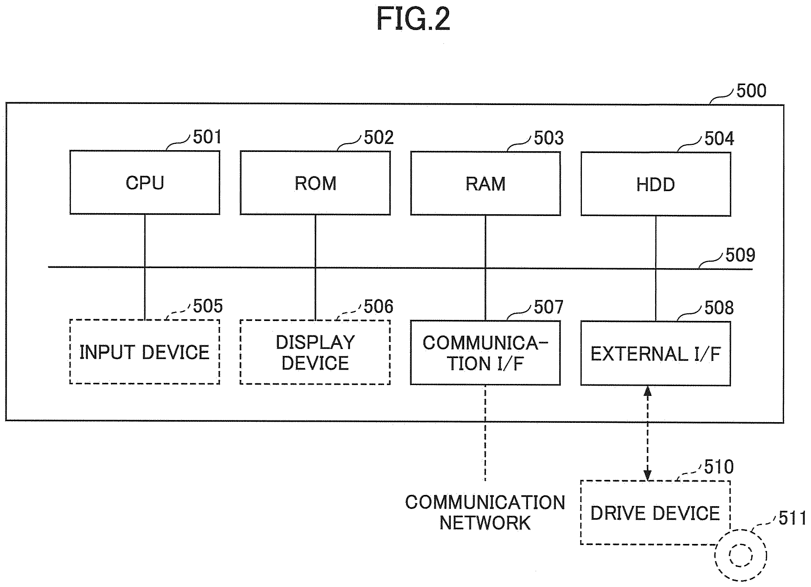

The hardware configuration of the design support system 1 in the present embodiment will be described with reference to FIG. 2.

### <Hardware Configuration of a Computer>

The material designing device 10 and the user terminal 20 of the present embodiment are realized by, for example, a computer. FIG. 2 is a block diagram showing an example of the hardware configuration of a computer 500 of the present embodiment.

As shown in FIG. 2, the computer 500 includes a CPU (Central Processing Unit) 501, a ROM (Read Only Memory) 502, a RAM (Random Access Memory) 503, an HDD (Hard Disk Drive) 504, an input device 505, a display device 506, a communication I/F (Interface) 507, and an external I/F 508. The CPU 501, the ROM 502, and the RAM 503 form the so-called computer. The pieces of hardware of the computer 500 are connected to each other via a bus line 509. The input device 505 and the display device 506 may be connected to the external I/F 508 for use.

The CPU 501 is an arithmetic unit that reads programs and data from a storage device such as the ROM 502 or the HDD 504 onto the RAM 503 and executes processing, thereby realizing the control and functions of the entire computer 500.

The ROM 502 is an example of a nonvolatile semiconductor memory (storage device) that can retain programs and data even when the power is turned off. The ROM 502 functions as a main storage device that stores various programs, data, and the like necessary for the CPU 501 to execute various programs installed in the HDD 504. Specifically, the ROM 502 stores boot programs, such as BIOS (Basic Input/Output System) and EFI (Extensible Firmware Interface), that are executed when the computer 500 is started, and data such as OS (Operating System) settings and network settings.

The RAM 503 is an example of a volatile semiconductor memory (storage device) that erases programs and data when the power is turned off. The RAM 503 is, for example, a DRAM (Dynamic Random Access Memory), a SRAM (Static Random Access Memory), or the like. The RAM 503 provides a work area that is expanded when various programs installed in the HDD 504 are executed by the CPU 501.

The HDD 504 is an example of a nonvolatile storage device that stores programs and data. The programs and data stored in the HDD 504 include the OS, which is the basic software that controls the entire computer 500, applications that provide various functions on the OS, and the like. The computer 500 may use a storage device (e.g., an SSD: Solid State Drive or the like) using a flash memory as a storage medium, instead of the HDD 504.

The input device 505 includes a touch panel used by the user to input various signals, operation keys and buttons, a keyboard and mouse, and a microphone to input sound data such as sound or the like.

The display device 506 includes a display such as liquid crystal or organic EL (ElectroLuminescence) for displaying a screen, a speaker for outputting sound data such as sound, and the like.

The communication I/F 507 is an interface for connecting to a communication network and enabling the computer 500 to perform data communication.

The external I/F 508 is an interface with an external device. The external device includes a drive device 510.

The drive unit 510 is a device for setting a recording medium 511. The recording medium 511 here includes a medium for recording information optically, electrically, or magnetically, such as a CD-ROM, a flexible disk, a magneto-optical disk, or the like. The recording medium 511 may also include a semiconductor memory or the like for recording information electrically on a ROM, flash memory, or the like. Thus, the computer 500 can read and/or write from or to the recording medium 511 via the external I/F 508.

Various programs installed on the HDD 504 are installed by, for example, setting the distributed recording medium 511 in the drive device 510 connected to the external I/F 508 and reading various programs, recorded on the recording medium 511, with the drive device 510. Alternatively, various programs installed on the HDD 504 may be installed by downloading these programs from another network different from the communication network, via the communication I/F 507.

### <Functional Configuration of the Design Support System>

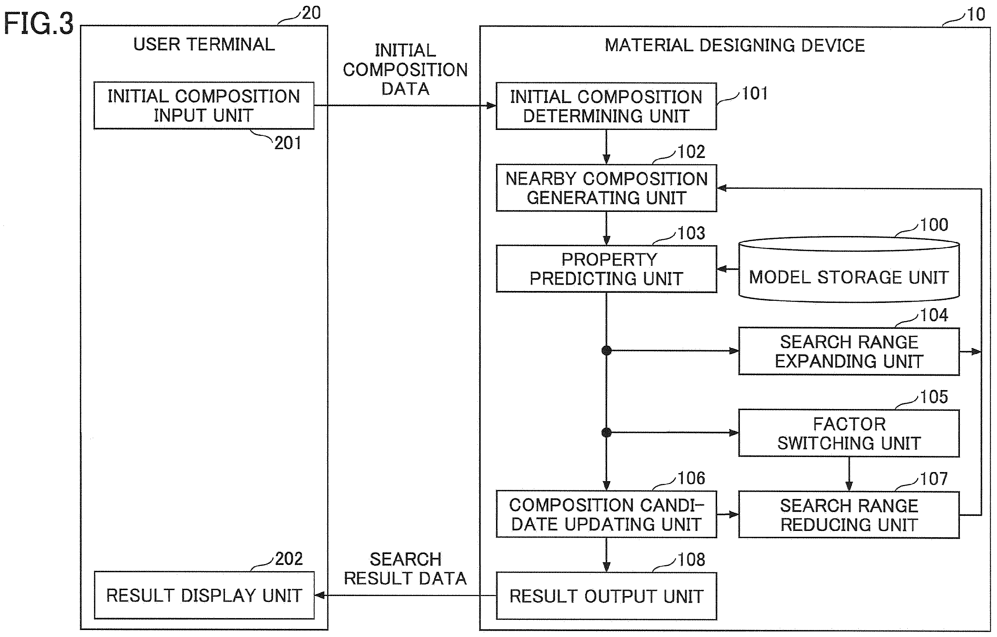

The functional configuration of the design support system in the present embodiment will be described with reference to FIG. 3. FIG. 3 is a block diagram showing an example of the functional configuration of the design support system 1 of the present embodiment.

### «Functional Configuration of the Material Designing Device»

As shown in FIG. 3, the material designing device 10 of the present embodiment includes a model storage unit 100, an initial composition determining unit 101, a nearby composition generating unit 102, a property predicting unit 103, a search range expanding unit 104, a factor switching unit 105, a composition candidate updating unit 106, a search range reducing unit 107, and a result output unit 108.

The initial composition determining unit 101, the nearby composition generating unit 102, the property predicting unit 103, the search range expanding unit 104, the factor switching unit 105, the composition candidate updating unit 106, the search range reducing unit 107, and the result output unit 108 are realized by causing the CPU 501 execute the program expanded on the RAM 503 from the HDD 504 shown in FIG. 2. The model storage unit 100 is realized by the HDD 504 shown in FIG. 2.

The model storage unit 100 stores the learned property prediction model. The property prediction model is a machine learning model in which each factor included in the material composition of the target substance is an explanatory variable and the material property of the target substance is an objective variable. An example of the machine learning model is a regression model. The structure of the machine learning model is, for example, a random forest, a deep neural network, or the like.

The initial composition determining unit 101 receives the initial composition data generated in response to the user operation, from the user terminal 20. The initial composition data in the present embodiment includes the material composition of the target substance and the target values of the material properties. The initial composition determining unit 101 determines the material composition of the target substance included in the initial composition data, as the initial value of the composition candidate.

The material composition to be included in the initial composition data should be selected, as a material composition having good measured values or predicted values for the desired material properties, from the experimental results or from the results of the large-scale search by machine learning. In addition, a material composition having good measured values for the desired material properties may be selected from the experimental results using the material composition searched for by the large-scale search. If a material composition with good material properties is selected from the experimental results as the initial value, it can be expected that a composition candidate with high feasibility can be obtained.

Whether or not the material properties are good indicates whether or not the product produced using the target substance should have excellent properties. When a larger value of the material property is superior as a product property, it can be said that the larger the value of the material property, the better the value. On the other hand, when a smaller value of the material property is superior as a product property, it can be said that the smaller the value of the material property, the better the value.

As an example, the experimental results using the material composition searched for by the large-scale search can be obtained as follows. First, a set of material compositions is generated in which each factor, from the entire material composition of the target substance, is varied within a predetermined variation width and across an entire range. Next, a predetermined number of material compositions are randomly sampled from the set of material compositions. Following this, experiments are performed on the sampled material compositions, and multiple material properties including desired material properties are measured. From the experimental results obtained in this way, a material composition having good desired material properties may be selected as a material composition to be included in the initial composition data. A predetermined number of material compositions may be sampled at a predetermined increment width from a set of material compositions.

The target values of material properties to be included in the initial composition data may be arbitrarily determined by the user. The best material properties obtained from the experimental results or the results of a large-scale search, or material properties further improved from the best material properties, may be determined as target values.

The nearby composition generating unit 102 generates a plurality of nearby compositions in which each factor included in the composition candidate of the target substance is varied within a predetermined search range. Specifically, the nearby composition generating unit 102 generates a predetermined number of nearby compositions by sampling a predetermined number of material compositions randomly or with a predetermined increment width, from a set of material compositions in which the mixture amount of each factor included in the composition candidate is varied within a predetermined variation width and a predetermined search range.

The property predicting unit 103 predicts a plurality of material properties based on each nearby composition generated by the nearby composition generating unit 102. The plurality of material properties include material properties to be maintained (hereinafter also referred to as "maintenance properties") and material properties to be improved (hereinafter also referred to as "improvement target properties"), among material properties based on the initial composition. The property predicting unit 103 calculates a plurality of material properties by inputting each factor included in the nearby composition into a learned property prediction model stored in the model storage unit 100.

The property predicting unit 103 may predict material properties for just the nearby compositions whose similarities with the composition candidate are equal to or greater than a predetermined threshold. The similarity in the present embodiment, for example, for each composition candidate and nearby composition, generates a vector using the mixture amount of each factor as an element, and calculates the distance between the vectors using an arbitrary distance scale. An example of the distance scale in the present embodiment is a Euclidean distance.

The search range expanding unit 104 expands the search range when there is no nearby composition whose predicted value of the material property, according to the property predicting unit 103, is better than the target value of the material property.

The factor switching unit 105 switches the factors of the composition candidate when the search range is equal to or greater than the predetermined maximum search range. One example of the maximum search range in the present embodiment is the range expanded a predetermined number of times by the search range expanding unit 104. The factor switching unit 105 returns the search range to the initial state when the factors are switched.

The factor switching unit 105 switches the factors based on the contribution of each factor in the learned property prediction model stored in the model storage unit 100. Specifically, in the initial value of the composition candidate, the factor switching unit 105 switches a factor having a high contribution to the improvement target property with a factor having a low contribution to the maintenance property.

When there is a nearby composition in which the predicted value of the material property, predicted by the property predicting unit 103, is better than the target value of the material property, the composition candidate updating unit 106 updates the composition candidate with this nearby composition. When the maintenance property of the nearby composition maintains the target value and the improvement target property of the nearby composition improves beyond the target value, the composition candidate updating unit 106 makes this nearby composition a new composition candidate. When the composition candidate is updated, the composition candidate updating unit 106 updates the target value of the material property with the predicted value of the material property that is based on the new composition candidate.

The maintenance of the material property means a case where no significant difference appears in the material property between the value of the original material property and the value of the new material property. The improvement of the material property means that the value of the new material property is better than the value of the original material property.

The search range reducing unit 107 reduces the search range to the initial state when the factor of the composition candidate is switched by the factor switching unit 105 or when the composition candidate is updated by the composition candidate updating unit 106.

When the predetermined end condition is satisfied, the result output unit 108 generates search result data and transmits it to the user terminal 20. One example of the end condition in the present embodiment is when the update of the composition candidate has been performed a predetermined number of times. Another example of the end condition in the present embodiment is when the predicted value of the material property based on the composition candidate achieves the target value designated by the user.

The search result data in the present embodiment includes the current composition candidate of the target substance and the predicted values of a plurality of material properties based on this composition candidate. The search result data in the present embodiment may include history information of composition candidates updated during the search. The history information of composition candidates may include, for example, a list of each composition candidate updated during the search and a list of predicted values of a plurality of material properties based on each composition candidate.

### <Functional Structure of User Terminal 20>

As shown in FIG. 3, the user terminal 20 in the present embodiment includes an initial composition input unit 201 and a result display unit 202.

The initial composition input unit 201 and the result display unit 202 are realized by processing performed by the CPU 501 executing a program expanded on the RAM 503 from the HDD 504 shown in FIG. 2.

The initial composition input unit 201 receives input of the initial composition data in response to a user operation. The initial composition input unit 201 transmits the received initial composition data to the material designing device 10.

The result display unit 202 receives search result data from the material designing device 10. Based on the received search result data, the result display unit 202 displays the composition candidate of the target substance and the material property of this composition candidate on the display device 506.

### <Processing Procedure of the Design Support System>

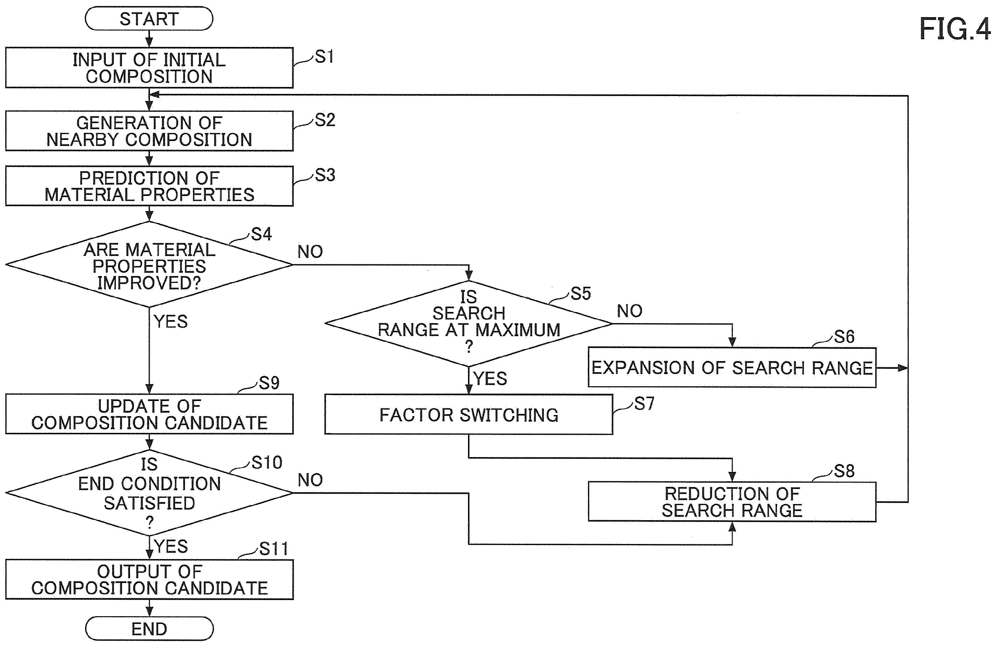

The processing procedure of the material designing method executed by the design support system 1 in the present embodiment will be described with reference to FIG. 4. FIG. 4 is a flowchart showing an example of the processing procedure of the material designing method in the present embodiment.

In step S1, the initial composition input unit 201 provided in the user terminal 20 receives the input of the initial composition data including the material composition of the target substance and the target value of this material property, in response to the operation of the user. Next, the initial composition input unit 201 transmits the received initial composition data to the material designing device 10.

In the material designing device 10, the initial composition determining unit 101 receives initial composition data from the user terminal 20. Next, the initial composition determining unit 101 determines the material composition of the target substance included in the initial composition data as the initial value of the composition candidate. Subsequently, the initial composition determining unit 101 sends the initial value of the composition candidate and the target value of the material property to the nearby composition generating unit 102.

In step S2, the nearby composition generating unit 102 provided in the material designing device 10 receives the initial value of the composition candidate and the target value of the material property from the initial composition determining unit 101. Next, the nearby composition generating unit 102 generates a plurality of nearby compositions in which each factor included in the composition candidate of the target substance is varied within a predetermined search range. Subsequently, the nearby composition generating unit 102 sends the generated plurality of nearby compositions to the property predicting unit 103.

As an example, the search range in the present embodiment is set to a range that is ±5% in the initial state. The search range is expanded to a range of ±10% in the first expansion. The search range is expanded to a range of ±15% in the second expansion.

In step S3, the property predicting unit 103 provided in the material designing device 10 receives a plurality of nearby compositions from the nearby composition generating unit 102. Next, the property predicting unit 103 calculates the similarity between the composition candidate and each nearby composition, and deletes each nearby composition whose similarity is less than a predetermined threshold value.

The property predicting unit 103 reads the learned property prediction model from the model storage unit 100. Next, the property predicting unit 103 inputs each factor included in each nearby composition into the learned property prediction model, thereby predicting a plurality of material properties including maintenance properties and improvement target properties.

In step S4, the property predicting unit 103 provided in the material designing device 10 determines whether there is a nearby composition whose predicted value of the material property is better than the target value of the material property, among the nearby compositions whose material properties have been predicted. If there is a nearby composition that improves the material property (YES), the property predicting unit 103 proceeds to the processing of step S9. On the other hand, if there is no nearby composition that improves the material property (NO), the property predicting unit 103 proceeds to the processing of step S5.

In step S5, the search range expanding unit 104 provided in the material designing device 10 determines whether the number of times expansion of the search range was performed is greater than or equal to a predetermined threshold value. If the number of times expansion was performed is greater than or equal to the predetermined threshold value (YES), the search range expanding unit 104 proceeds to the processing of step S7. On the other hand, if the number of times expansion was performed is less than the predetermined threshold value (NO), the search range expanding unit 104 proceeds to the processing of step S6.

In the present embodiment, the maximum number of times expansion is performed is two. That is, if the current search range is ±15% or more, the search range expanding unit 104 proceeds to the processing of step S7. If the current search range is less than ±15% (±5% or ±10%), the search range expanding unit 104 proceeds to the processing of step S6.

In step S6, the search range expanding unit 104 provided in the material designing device 10 expands the search range. The search range expanding unit 104 expands the search range to ±10% if the current search range is in a range of ±5%, and expands the search range to ±15% if the current search range is in a range of ±10%.

In step S7, the factor switching unit 105 provided in the material designing device 10 reads the learned property prediction model from the model storage unit 100. Next, the factor switching unit 105 acquires the contribution of each factor from the learned property prediction model. The contribution is an index representing the magnitude of the influence of each factor on the model learning. The factor switching unit 105 acquires the contribution of each factor for each material property predicted by the property predicting unit 103.

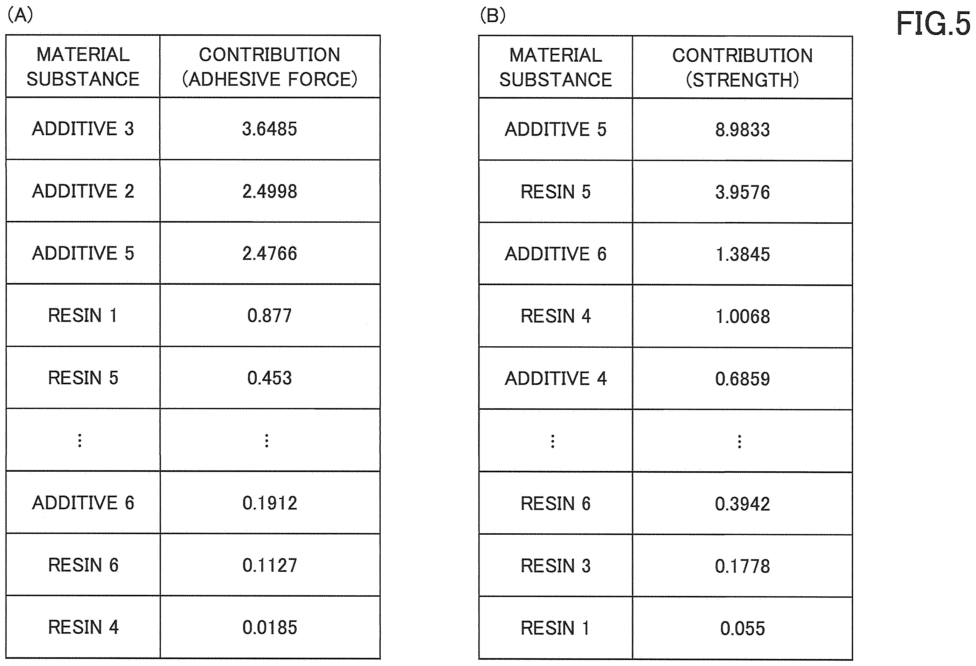

Here, the contribution in the present embodiment will be described with reference to FIG. 5. FIG. 5 shows an example of the contribution in the present embodiment.

FIG. 5 assumes a case where the target substance is an adhesive and the strength is to be improved while maintaining the adhesive force that is based on the initial value of the composition candidate. Fig. 5(A) shows an example of the contribution to the adhesive force. Fig. 5(B) shows an example of the contribution to the strength.

As shown in Fig. 5, the contribution of each factor is a combination of the factor and the contribution, and is obtained for each material property. The example shown in Fig. 5 is an example of the contribution obtained from the property prediction model implemented by a random forest. However, if an index representing the influence on learning or prediction can be obtained or derived from the learned model, this index may be used as the contribution.

The factor switching unit 105 switches the factor of the composition candidate based on the contribution of each factor. Specifically, the factor switching unit 105 switches a factor having a high contribution to the improvement target property with a factor having a low contribution to the maintenance property. It should be noted that the factor switching unit 105 switches factors of the same type of material substance with each other. For example, when the factor having a high contribution to the improvement target property is an additive, a factor having a low contribution to the maintenance property is selected among the additives.

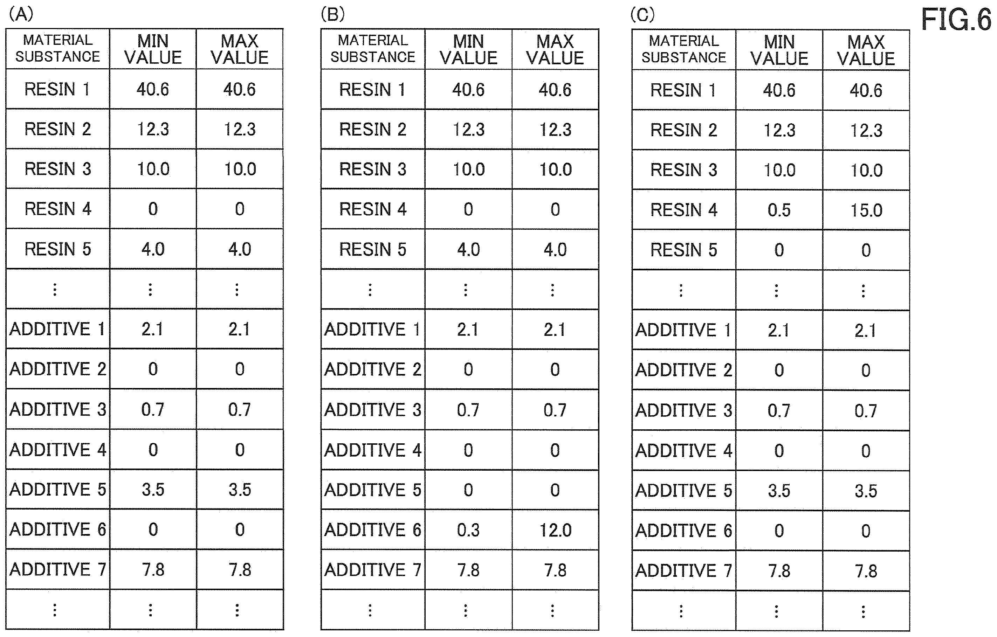

Here, the factor switching in the present embodiment will be described with reference to FIG. 6. FIG. 6 is a diagram showing an example of the composition candidates before and after the factor switching in the present embodiment.

FIG. 6(A) is a diagram showing examples of the initial values of the composition candidates. FIG. 6(B) is a diagram showing examples of the composition candidates after the first factor switching. FIG. 6(C) is a diagram showing examples of the composition candidates after the second factor switching.

As shown in FIG. 6, the minimum and maximum values are set for the factors after factor switching. The minimum and maximum values represent the minimum and maximum amounts that can be mixed with the material substance in the production of the target substances. The minimum and maximum values are set in advance based on the results of other experiments using the material substances.

In the example of the first factor switching shown in FIG. 6(B), it is understood that "Additive 5" is switched to "Additive 6". Among the material substances included in the composition candidates shown in FIG. 6(A), "Additive 5" has the highest contribution to the strength shown in FIG. 5(B). Among the additives not included in the composition candidates shown in FIG. 6(A), "Additive 6" has the lowest contribution to the adhesive force shown in FIG. 5(A). Therefore, in the example of the factor switching shown in FIG. 6, "Additive 5" is switched to "Additive 6".

In the factor switching, the factor switching is always performed based on the initial value of the composition candidate. That is, even if factor switching is performed multiple times, no further factor switching is performed from the composition candidate after the factor switching. Thus, the material composition obtained as a search result is obtained by switching only one factor with the initial value of the composition candidate, and the initial value dependence can be maintained.

In the example of the second factor switching shown in FIG. 6(C), it can be understood that "Resin 5" is switched with "Resin 4." Among the material substances included in the composition candidates shown in FIG. 6(A), "Resin 5" has the highest contribution to the strength shown in FIG. 5(B), except for "Additive 5", and among the resins not included in the composition candidates shown in FIG. 6(A), "Resin 4" has the lowest contribution to the adhesive force shown in FIG. 5(A). At this time, it can be understood that "Additive 5" and "Additive 6" switched in the first factor switching have returned to their initial values.

Referring back to FIG. 4, a description will be provided. In step S8, the search range reducing unit 107 provided in the material designing device 10 reduces the search range to the initial state. If the current search range is a range exceeding ±5% (±10% or ±15%), the search range reducing unit 107 reduces the search range to a range of ±5%.

In step S9, when there is a nearby composition in which the predicted value of the material property is better than the target value of the material property, the composition candidate updating unit 106 provided in the material designing device 10 updates the composition candidate with this nearby composition. When there are a plurality of nearby compositions in which the predicted value of the material property is better than the target value of the material property, the composition candidate updating unit 106 updates the composition candidate with the nearby composition having the best material property among these nearby compositions. Next, the composition candidate updating unit 106 updates the target value of the material property with the predicted value of the material property that is based on the new composition candidate.

In step S10, the composition candidate updating unit 106 provided in the material designing device 10 determines whether the number of times of updating the composition candidate is equal to or greater than a predetermined threshold value. If the number of times of updating is equal to or greater than the predetermined threshold value (YES), the composition candidate updating unit 106 proceeds to the processing of step S11. On the other hand, if the number of times of updating is less than the predetermined threshold value (NO), the composition candidate updating unit 106 returns to the processing of step S8.

An example of the predetermined number of times in the present embodiment is five times. If the number of times the composition candidate is updated is too many, the search may occur in a region away from the initial value of the composition candidate. As a result, problems such as loss of initial value dependency, output of material compositions that are not realistic, and the like occur. Therefore, in the present embodiment, the number of times the composition candidate is updated is limited, and the search is terminated after a predetermined number of updates are performed.

In step S11, the result output unit 108 provided in the material designing device 10 generates search result data including the composition candidate of the target substance and the predicted value of the material property that is based on this composition candidate. Next, the result output unit 108 transmits the search result data to the user terminal 20.

In the user terminal 20, the result display unit 202 receives the search result data from the material designing device 10. The result display unit 202 displays the composition candidate of the target substance and the material property in this composition candidate on the display device 506, based on the received search result data.

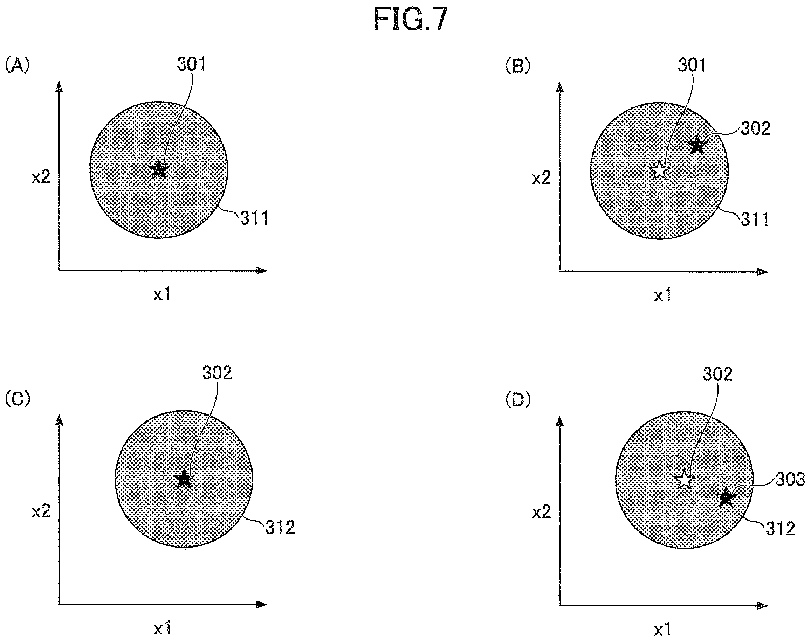

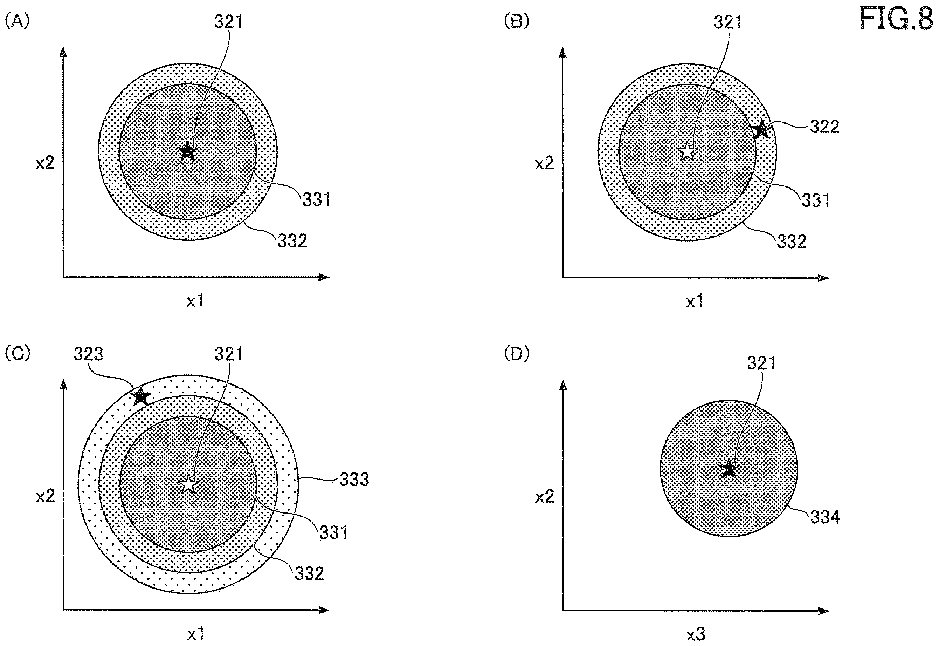

Here, the flow of the search in the present embodiment will be described with reference to FIGS. 7 and 8. FIGS. 7 and 8 show an example of the flow of the search.

The plane shown in FIG. 7 is a graph representing the distribution of the material composition. Actually, it should be expressed in a space having the same number of dimensions as the number of factors included in the material composition, but for the purpose of explanation, it is expressed in a two-dimensional plane using two arbitrary factors x1 and x2.

FIG. 7(A) shows that, in the first local search, the local search is performed within the search range 311 that is ±5% from the initial value 301 of the composition candidate. FIG. 7(B) shows that, as a result of the first local search, a new composition candidate 302 is searched for within the search range 311 of ±5%.

FIG. 7(C) shows that, in the second local search, the local search is performed within the search range 312 of ±5% from the new composition candidate 302. FIG. 7(D) shows that, as a result of the second local search, a new composition candidate 303 is searched for within the ±5% search range 312.

FIG. 8(A) shows that, in the x-th local search, the composition candidate is not updated within the ±5% search range 331 from the composition candidate 321, and the local search is performed within the ±10% search range 332. FIG. 8(B) shows that, as a result of the x-th local search, a new composition candidate 322 is searched for within the ±10% search range 332.

FIG. 8(C) shows that, in the x-th local search, the composition candidate is not updated within the ±10% search range 332 from the composition candidate 321, and the local search is performed within the ±15% search range 333. As a result, a new composition candidate 323 is searched for.

FIG. 8(D) shows that, in the x-th local search, the composition candidate is not updated within the ±15% search range 333 from the composition candidate 321, and the factor x1 is switched to a factor x3. At this time, the search range is reduced to the ±5% search range 334 from the composition candidate 321, and the local search is performed within the search range 334.

### <Embodiment>

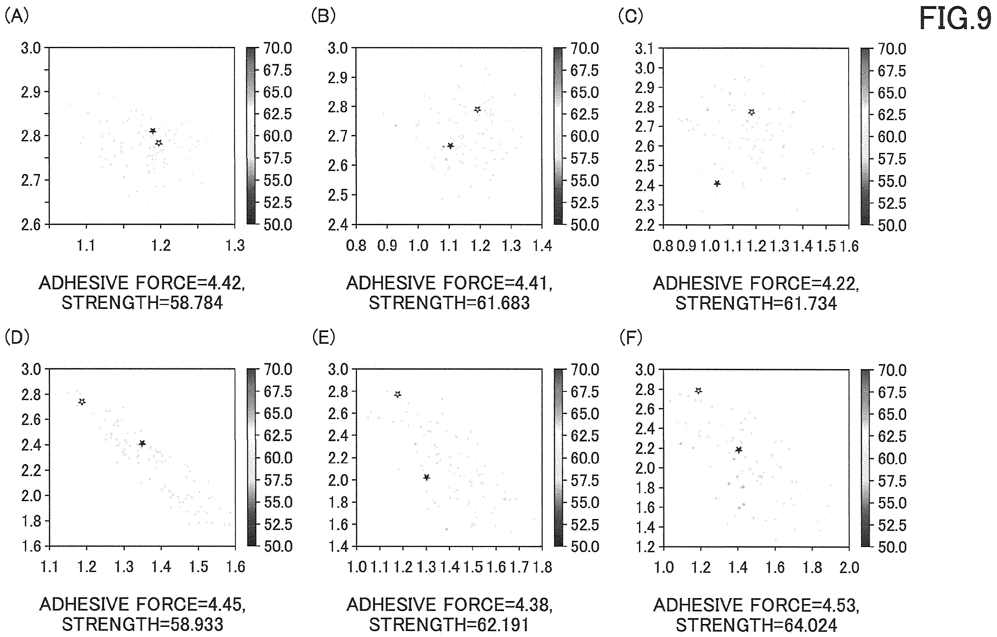

One embodiment of the present disclosure is an example of searching for a material composition that can obtain the strength that achieves the target value while maintaining the adhesive force, in the material design of an adhesive, with the material designing device 10 in the present embodiment. FIGS. 9 to 11 are diagrams showing examples of the search results of the material composition in the present embodiment.

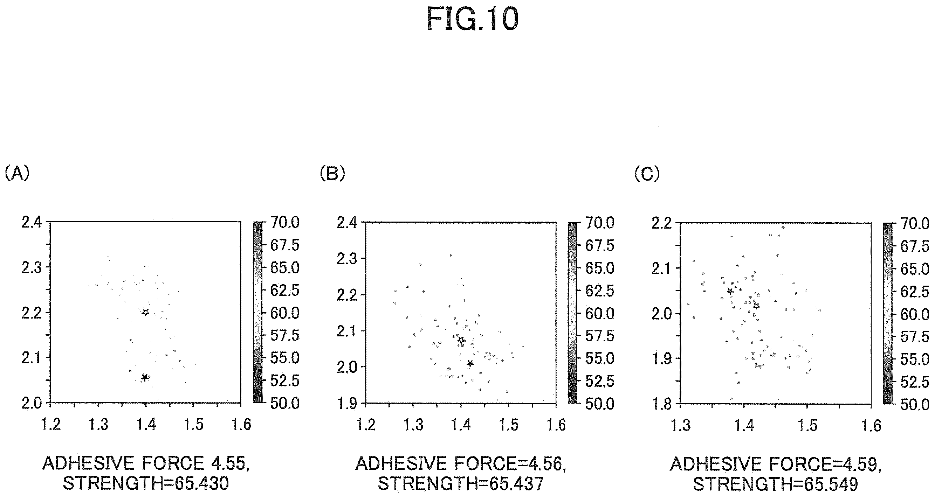

In the present embodiment, a material composition that obtained the adhesive force of 4.4 and the strength of 52 was selected as the initial value of the composition candidate. For this, the target value of the adhesive force was set to 4.6 and the target value of the strength was set to 64. The graphs shown in FIGS. 9 and 10 each show the coordinates and the material properties in the search space, for a nearby composition generated by one local search. In each graph shown in FIGS. 9 and 10, the left vertical axis and the horizontal axis show the material composition, and the right vertical axis shows a color bar in which the color indicates the strength, which is the target property to be improved. In each graph, the initial value is indicated by a star mark that is white, and the best value is indicated by a star mark that is black.

FIG. 9(A) shows the results of the first local search. In the first local search, the nearby composition was generated in the range of ±5%. The best values were 4.42 for the adhesive force and 58.784 for the strength. The candidate composition was not updated, because the material properties did not meet the target values.

FIG. 9(B) shows the results of the second local search. In the second local search, the nearby composition was generated in the range of ±10%. The best values were 4.41 for the adhesive force and 61.683 for the strength. The composition candidate was not updated, because the material properties did not meet the target values.

FIG. 9(C) shows the results of the third local search. In the third local search, the nearby composition was generated in the range of ±15%. The best values were 4.22 for the adhesive force and 61.734 for the strength. The candidate composition was not updated, because the material properties did not meet the target values.

FIG. 9(D) shows the results of the first local search after the factor switching. In the first local search after the factor switching, the nearby composition was generated in the range of ±5%. The best values were 4.45 for the adhesive force and 58.933 for the strength. The composition candidate was not updated, because the material properties did not meet the target values.

FIG. 9(E) shows the results of the second local search after the factor switching. In the second local search after the factor switching, the nearby composition was generated in the range of ±10%. The best values were 4.38 for the adhesive force and 62.191 for the strength. The composition candidate was not updated, because the material properties did not meet the target values.

Fig. 9(F) shows the results of the third local search after the factor switching. In the third local search after the factor switching, the nearby composition was generated in the range of ±15%. The best values were 4.53 for the adhesive force and 64.024 for the strength. Since the material properties satisfied a target value, the composition candidate and target values were updated with the material composition in which the best values were obtained.

Fig. 10(A) shows the results of the first local search after the first update. In the first local search after the first update, the nearby composition was generated in the range of ±5%. The best values were 4.55 for the adhesive force and 65.430 for the strength. Since the material properties met the target values, the second update was performed with the material composition that obtained the best values.

Fig. 10(B) shows the results of the first local search after the second update. The first local search after the second update generated a nearby composition in the range of ±5%. The best values were 4.56 for the adhesive force and 65.437 for the strength. Since the material properties satisfied the target values, the third update was performed with the material composition that obtained the best values.

Fig. 10(C) shows the results of the first local search after the third update. In the first local search after the third update, the nearby composition was generated in the range of ±5%. The best values were 4.59 for the adhesive force and 65.549 for the strength. Since the material properties satisfied the target values, the fourth update was performed with the material composition that obtained the best values.

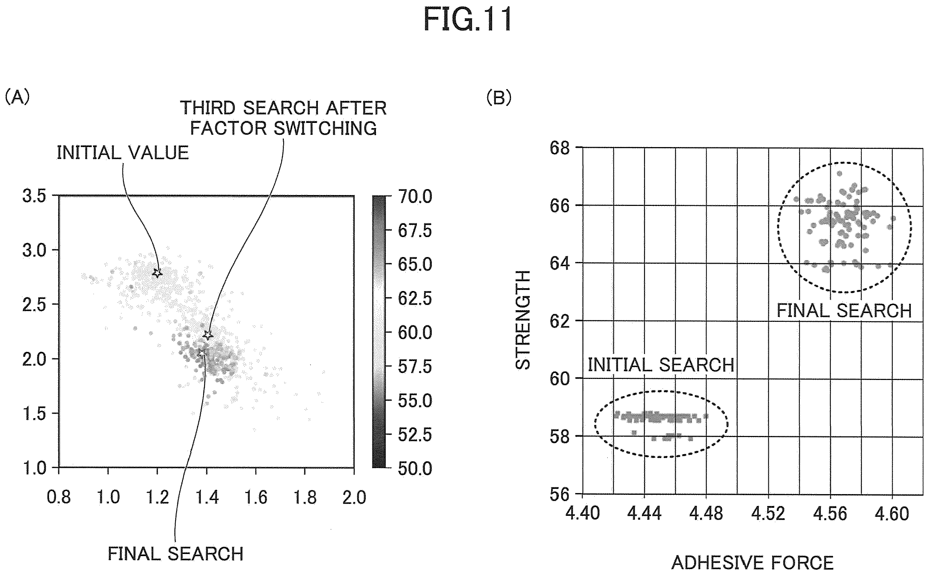

FIG. 11 shows an example of the search results of the material composition in the present embodiment. FIG. 11(A) is a graph summarizing the search results from the first local search (hereinafter referred to as "initial search") to the first local search after the third update (hereinafter referred to as "final search") shown in FIGS. 9(A) to 10(C). The graph shown in FIG. 11(A) shows the initial value in the initial search, the best value in the third search after the factor switching, and the best value in the final search. As shown in FIG. 11(A), it is understood that in the search where the material composition that achieved a target value was obtained, the search was performed in a close region.

FIG. 11(B) is a graph that plots the material properties of the nearby composition in the initial search and the material properties of the nearby composition in the final search, where the horizontal axis is the adhesive force and the vertical axis is the strength. As shown in FIG. 11(B), it is understood that, in the final search, the search was carried out in a region where the adhesive force and the strength were higher than in the first search.

From the above results, it was shown that the material designing device 10 of the present embodiment can efficiently search for a material composition that can obtain good material properties.

### <Effect of the Embodiment>

The design support system of the present embodiment updates the composition candidate with a nearby composition that improves the material properties, when there is a nearby composition that improves the material properties among the nearby compositions in which each factor included in the composition candidate of the target substance is varied within a predetermined search range, and expands the search range when there is no nearby composition that improves the material properties. At this time, when the search range exceeds the predetermined range, the factors of the composition candidate are switched. Therefore, according to the design support system of the present embodiment, it is possible to perform a local search that is unlikely to fall into a local solution, while maintaining the initial value dependency.

In particular, in the local search, the design support system of the present embodiment maintains the material property desired to be maintained, and updates the composition candidate with the nearby composition that improves the material property desired to be improved. In the factor switching, the factor with a high contribution to the material property desired to be improved is switched with a factor with a low contribution to the material property desired to be maintained. Therefore, according to the design support system of the present embodiment, it is possible to search for a material composition that improves the material property, while considering a plurality of material properties in a trade-off relationship.

Furthermore, the design support system of the present embodiment updates the composition candidate a predetermined number of times, then ends the search and outputs this composition candidate as the search result. Furthermore, the design support system of the present embodiment does not search for the nearby composition whose similarity with the composition candidate is less than a predetermined threshold value. Therefore, according to the design support system of the present embodiment, the composition candidate is not searched for in a region away from the initial value of the composition candidate, and the initial value dependency can be maintained.

### [Note]

Each function of the above-described embodiment can be realized by one or more processing circuits. Here, the term "processing circuit" in this specification includes a processor programmed to execute each function by software, such as a processor implemented by an electronic circuit, or equipment such as an ASIC (Application Specific Integrated Circuit), DSP (Digital Signal Processor), FPGA (Field Programmable Gate Array), or conventional circuit module designed to execute each function described above.

Although the embodiments of the present invention have been described in detail above, the present invention is not limited to these embodiments and can be modified or changed in various ways within the scope of the gist of the present invention as described in the claims.

This application claims priority on Japanese Patent Application No. 2022-130049, filed with the Japan Patent Office on August 17, 2022, and is incorporated herein by reference in its entirety.

### [Description of Reference Symbols]

- 1: Design support system
- 10: Material designing device
- 101: Initial composition determining unit
- 102: Nearby composition generating unit
- 103: Property predicting unit
- 104: Search range expanding unit
- 105: Factor switching unit
- 106: Composition candidate updating unit
- 107: Search range reducing unit
- 108: Result output unit
- 20: User terminal
- 201: Initial composition input unit
- 202: Result display unit

## Claims

1. A material designing device comprising:
a nearby composition generating unit configured to generate a nearby composition in which each factor included in a composition candidate of a target substance is varied within a predetermined search range;
a property predicting unit configured to predict a material property of the target substance based on the nearby composition;
a composition candidate updating unit configured to update the composition candidate with the nearby composition, when there is the nearby composition which improves the material property;
a search range expanding unit configured to expand the search range, when there is no nearby composition which improves the material property; and
a factor switching unit configured to switch a factor of the composition candidate, when the search range exceeds a predetermined maximum search range.

2. The material designing device according to claim 1, wherein
the property predicting unit is configured to predict the material property by using a prediction model in which the material composition of the target substance is an explanatory variable and the material property is an objective variable, and
the factor switching unit is configured to switch factors of the composition candidate based on a contribution of each factor in the prediction model.

3. The material designing device according to claim 2, wherein
the property predicting unit is configured to predict a plurality of material properties, and
the composition candidate updating unit is configured to update the composition candidate when a first material property is maintained and a second material property is improved.

4. The material designing device according to claim 3, wherein
the factor switching unit is configured to switch a factor having a high contribution to the second material property with a factor having a low contribution to the first material property.

5. The material designing device according to claim 4, further comprising:
an initial composition determining unit configured to determine the material composition having a good first material property as the composition candidate, among material compositions generated randomly or with a predetermined increment size from all material compositions of the target substance.

6. The material designing device according to claim 4 or 5, wherein
the factor switching unit is configured to switch factors of a same type.

7. The material designing device according to any one of claims 1 to 6, wherein
the composition candidate updating unit is configured to output the composition candidate, when the composition candidate has been updated a predetermined number of times.

8. The material designing device according to any one of claims 1 to 7, wherein
the property predicting unit is configured to predict the material property for the nearby composition having a similarity with the composition candidate equal to or greater than a predetermined threshold value.

9. A material designing method in which a computer executes the following:
a procedure of generating a nearby composition in which each factor included in a composition candidate of a target substance is varied within a predetermined search range;
a procedure of predicting a material property of the target substance based on the nearby composition;
a procedure of updating the composition candidate with the nearby composition, when there is the nearby composition which improves the material property;
a procedure of expanding the search range when there is no nearby composition which improves the material property; and
a procedure of switching a factor of the composition candidate, when the search range exceeds a predetermined maximum search range.

10. A program causing a computer to execute the following:
a procedure of generating a nearby composition in which each factor included in a composition candidate of a target substance is varied within a predetermined search range;
a procedure of predicting a material property of the target substance based on the nearby composition;
a procedure of updating the composition candidate with the nearby composition, when there is the nearby composition which improves the material property;
a procedure of expanding the search range when there is no nearby composition which improves the material property; and
a procedure of switching a factor of the composition candidate, when the search range exceeds a predetermined maximum search range.
